# EUROPEAN PATENT APPLICATION

(11) **EP 3 785 731 A1**
(43) Date of publication of application: **03.03.2021**
(21) Application number: 19194037.8
(22) Date of filing: 28.08.2019
(51) Int. Cl.: A61K 39/395, C07K 16/28, A61K 39/00

(54) **TREATING NON-ALCOHOLIC STEATOHEPATITIS (NASH) AND HEPATOCELLULAR CARCINOMA (HCC) WITH COMPOUNDS BINDING THE ECTODOMAIN OF PLATELET GLYCOPROTEIN IB (GPIB) ALPHA**

(71) Applicant: Deutsches Krebsforschungszentrum, Stiftung des öffentlichen Rechts, 69120 Heidelberg (DE)
(72) Inventor: The designation of the inventor has not yet been filed
(74) Representative: Kuttenkeuler, David

(57) **Abstract**

The disclosure is based on the finding that a specific binding of compounds to the ectodomain of platelet glycoprotein Ib (GPIb) alpha reduces the occurrence and progression of non-alcoholic fatty liver disease (NAFLD) or non-alcoholic steatohepatitis (NASH), or of a disorder or condition associated with NAFLD or NASH, such as a disorder or condition developing from NAFLD or NASH (such as Hepatocellular Carcinoma). The disclosure provides treatment options of NAFLD/NASH and HCC patients based on the specific binding to the GPIb ectodomain, and preferably by impairing GPIb-thrombin interaction. The disclosure further proposes medical treatments as well screening methods for the identification of compounds suitable for the treatment of NAFLD/NASH and HCC.

## Description

### FIELD OF THE INVENTION

The invention is based on the finding that a specific binding of compounds to the ectodomain of platelet glycoprotein Ib (GPIb) alpha reduces the occurrence and progression of non-alcoholic fatty liver disease (NAFLD) or non-alcoholic steatohepatitis (NASH), or of a disorder or condition associated with NAFLD or NASH, such as a disorder or condition developing from NAFLD or NASH (such as Hepatocellular Carcinoma). The present invention provides new treatment options of NAFLD/NASH and HCC patients based on the specific binding to the GPIb ectodomain, and preferably by impairing GPIb-thrombin interaction. The invention provides medical treatments as well screening methods for the identification of compounds suitable for the treatment of NAFLD/NASH and HCC.

### DESCRIPTION

Changes in our lifestyle and diet have dramatically increased the incidence of obesity, overweight and metabolic syndrome. Overweight and obesity are not only a problem in the developed countries of the Western world. Meanwhile, children and adults in developing countries are also strongly affected as well (Anstee et al., 2019). The latest WHO report predicts that the number of obese diseases will double to triple over the next two decades (Stewart and Wild, 2014).

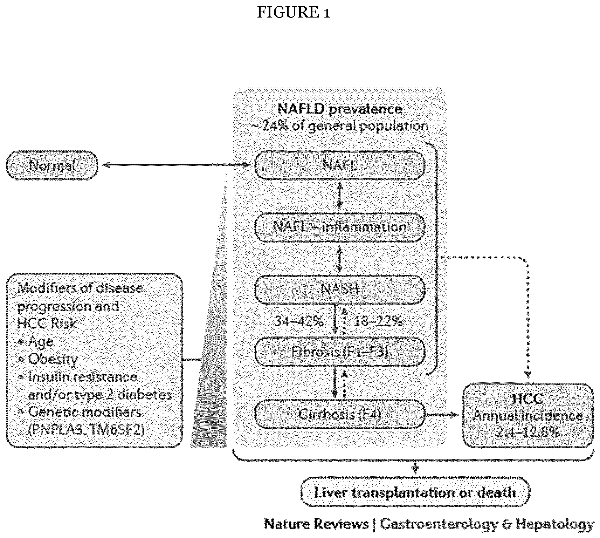

The liver, the body's most important metabolic organ, is severely affected by chronic hypercaloric intake, obesity, sedentary lifestyle and the resulting pathologies. The most common liver disease worldwide, non-alcohol fatty liver disease (NAFLD), is a clinical manifestation of overweight/obesity and metabolic syndrome (Figure 1). NAFLD is a chronic disease that can last up to several decades. NAFLD is characterized by predominant macrovesicular steatosis and metabolic deterioration of the liver, but not necessarily by obvious clinical symptoms. Currently, 90 million Americans and 40 million Europeans have NAFLD (Ringelhan et al., 2018; Anstee et al., 2019). The close link between obesity and cancer incidence is well known (Calle and Kaaks, 2004). In fact, a significant number of NAFLD patients develop non-alcoholic steatohepatitis (NASH) - a pathological from of fatty liver disease, which predisposes to fibrosis and hepatocellular carcinoma (HCC) (Anstee et al., 2019) - or liver dysfunction.

The number of people with NAFLD and NASH is steadily increasing in the USA and Europe (Loomba et al., 2013; Ringelhan et al., 2018; Anstee et al., 2019). Therefore, obesity, steatosis and steatohepatitis, which are the causes of the increasing incidence of liver dysfunction and HCC, are the main causes of attention in Western countries (White et al., 2012).

Until now, chronic viral infections (e.g. hepatitis B and C viruses) have been the most widespread etiology leading to the development of liver dysfunction and HCC in industrialized countries. However, the number of patients with NAFLD is increasing on a daily basis - and thus NAFLD has become the most prevalent liver disease in developed and developing countries. Consequently, HCC is the cancer with the highest growth rate of patients in the United States and a similar trend is already observed in Europe (Anstee et al., 2019).

At the same time, there are no efficient therapeutics to treat NASH and the treatment options for late-stage NASH-induced HCC are limited (Villanueva et al., 2014; Ringelhan et al., 2018; Anstee et al., 2019). This is mainly due to the fact that until recently the most important mechanisms triggering this chronic inflammatory/metabolic liver disease have not been understood. As a consequence, specific therapeutic treatments are urgently needed that do not cause side effects.

In the past decade, several models have been established to mimic NASH and NASH to HCC transition. On the one hand, these models have been genetically driven and on the other hand, these models have been triggered through distinct dietary origin (Anstee et al., 2019). Importantly, most of the pre-clinical models used so far (genetic and dietary) have failed to phenocopy the chronicity of the disease as well as the concomitant existence of co-lateral diseases that are found with NASH: type 2 diabetes, abdominal obesity, NASH related liver pathology - the chronic presence of the metabolic syndrome.

Moreover, many of the models contain genetic aberrations that on one hand, induce high NASH and HCC incidence and fast disease development, which does not necessarily reflect the pathophysiological dynamics of NASH, a disease that develops slowly and lasts several decades. In C57BL/6 mice, NASH can be induced by methionine/choline-deficient diet (MCD) or choline-deficient diet (CD) but not by high fat diet (HFD) alone (Hebbard and George, 2011). However, C57BL/6 mice fed with MCD or CD do not develop obesity, metabolic syndrome or HCC and the diet has to be discontinued after a few months due to weight loss (up to 40%) or cachexia (Hebbard and George, 2011). Hence, these short-term approaches fail to recapitulate NASH-induced long-term consequences found in the human liver and possibly other metabolic organs.

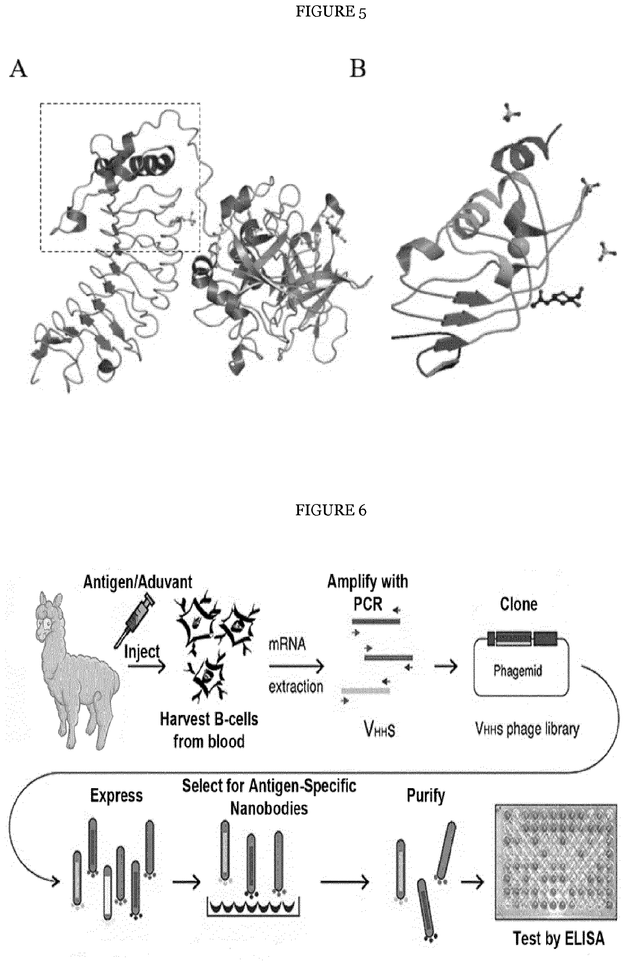

Due to the lack of appropriate mouse models, the exact long-term mechanisms leading to NASH and HCC have remained largely unknown. Deficiency of the essential nutrient choline was reported in NAFLD patients to exacerbate NAFLD and NASH (Guerrerio et al., 2012). Moreover, humans with insufficient choline uptake have defects in hepatic lipoprotein secretion, oxidative damage caused by mitochondrial dysfunction, and ER stress (Corbin and Zeisel, 2012). These clinical observations formed the basis for the present invention (Wolf et al., Cancer Cell, 2014): The inventors combined choline deficiency with a high fat diet (CD-HFD) or with a Western diet (WD) (High fat; fructose; cholesterol) for 12 months and thereby established a model in C57BL/6 mice, leading to metabolic syndrome, chronic NASH and NASH-driven HCC. Conflicting results have been published in the past using short-term in vivo experiments on the role of immune cells in experimentally induced NASH (Martin-Murphy et al., 2014; Lynch et al., 2012; Bhattacharjee et al., 2014). In contrast, recent data indicate for the first time that immune cells play an important role in triggering steatosis, NASH and NASH-driven HCC (Wolf et al., Cancer Cell, 2014), based on a long-term CD-HFD or WD leading to metabolic syndrome in C57BL/6 mice (Mahlemir et al., Nature Medicine 2019; incorporated herein by reference in its entirety, in particular the descriptions and results shown in figure 6 therein).

Thus, it is an objection of the invention to provide new treatment options for NAFLD/NASH and eventually HCC patients.

### BRIEF DESCRIPTION OF THE INVENTION

Generally, and by way of brief description, the main aspects of the present invention can be described as follows:

In **a first aspect,** the invention pertains to a compound for use in the treatment of non-alcoholic fatty liver disease (NAFLD) or non-alcoholic steatohepatitis (NASH), or of a disorder or condition associated with NAFLD or NASH, such as a disorder or condition developing from NAFLD or NASH (such as Hepatocellular Carcinoma), wherein the compound specifically binds to an ectodomain of platelet glycoprotein Ib (GPIb).

In **a second aspect,** the invention pertains to a pharmaceutical composition comprising a compound according to the first aspect.

In **a third aspect,** the invention pertains to a method for producing an antibody, antibody-like molecule, or antigenic binding fragment thereof, comprising the steps of: immunizing a non-human animal with a peptide, the peptide comprising, or consisting of, a sequence of the the ectodomain of GPIb, and isolating from that immunized mammal an immune cell expressing such antibody, antibody-like molecule or antigenic binding fragment thereof.

In **a fourth aspect,** the invention pertains a method for identifying a compound suitable for the treatment of non-alcoholic fatty liver disease (NAFLD) or non-alcoholic steatohepatitis (NASH), or of a disorder or condition associated with NAFLD or NASH, such as a disorder or condition developing from NAFLD or NASH, the method comprising the steps of
**(a)** Providing (x) a first cell expressing a protein or mRNA of GPIb, or of a variant of GPIb, or providing (y) a first test protein comprising, or (essentially) consisting of, an ectodomain of GPIb, or of a variant of GPIb,
**(b)** Providing a candidate compound,
**(c)** Bringing into contact the first cell or first test protein and the candidate compound, and
**(d)** Determining subsequent to step (c), either or both of:
   **(i)** A binding of the candidate compound to the first cell or to the first test protein; and/or
   **(ii)** A binding of the candidate compound to first cell or first test protein which binding is competitive with a thrombin protein;
wherein a binding in (i) or a competitive binding in (ii) indicates the candidate compound as suitable for the treatment of non-alcoholic fatty liver disease (NAFLD) or non-alcoholic steatohepatitis (NASH), or of a disorder or condition associated with NAFLD or NASH, such as a disorder or condition developing from NAFLD or NASH

In **a fifth aspect,** the invention pertains to a method for the treatment of non-alcoholic fatty liver disease (NAFLD) or non-alcoholic steatohepatitis (NASH), or of a disorder or condition associated with NAFLD or NASH, such as a disorder or condition developing from NAFLD or NASH, wherein the treatment comprises a step of administering to a subject in need of the treatment a therapeutically effective amount of a compound which specifically binds to an ectodomain of platelet glycoprotein Ib (GPIb).

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In **a first aspect,** the invention pertains to a compound for use in the treatment of non-alcoholic fatty liver disease (NAFLD) or non-alcoholic steatohepatitis (NASH), or of a disorder or condition associated with NAFLD or NASH, such as a disorder or condition developing from NAFLD or NASH (such as Hepatocellular Carcinoma), wherein the compound specifically binds to an ectodomain of platelet glycoprotein Ib (GPIb).

Hence, the present invention in particular pertains to the medical application of GPIb ectodomain binding via various compounds for the treatment of liver disorders such as NAFLD, NASH and/or HCC. In this first aspect it shall be understood that phrases such as "compound for use in a treatment" equally discloses as alternative embodiments the corresponding "methods for treating a disorder by using a compound", and "use of a compound for the manufacture of a medicament for the treatment of a disorder".

Therefore, the invention in the first aspect pertains also to a compound for use in the treatment of non-alcoholic fatty liver disease (NAFLD) or non-alcoholic steatohepatitis (NASH), or of a disorder or condition associated with NAFLD or NASH, such as a disorder or condition developing from NAFLD or NASH (such as Hepatocellular Carcinoma), wherein the compound is a modulator of platelet glycoprotein Ib (GPIb) (α) binding/interaction to thrombin, preferably to α-thrombin.

The term "platelet glycoprotein Ib (GPIb)" shall gernally refer to a membrane located glycoprotein found on platelets. Preferred is platelet glycoprotein Ib (GPIb) alpha chain (GPIbα), and most preferably human GPIbα. Human GPIbα is a protein that is expressed by the gene *glycoprotein Ib platelet subunit alpha* chromosomally located on 17p13.2. The gene can be found under the accession number HGNC:4439 (The HUGO Gene Nomenclature Committee at the European Bioinformatics Institute; https://www.genenames.org/). The protein of GPIbα is shown as amino acid sequence in SEQ ID NO: 1 and can be identified in the UniProt database with the accession P07359 (https://www.uniprot.org/uniprot/P07359; Nucleic Acids Res. 47: D506-515 (2019)).

Furthermore, the term "platelet glycoprotein Ib (GPIb)" in context of the invention may refer to variants or fragments of human GPIbα. Such variants or fragments may be orthologs, homologs or paralogs of human GPIbα. Alternatively such variants or fragments are proteins having an amino acid sequence that is at least 50, 60, 70, 80, 85, 90, 95, 96, 97, 98, 99, or 100% identical to the sequence of the ectodomain of human GPIbα, preferably of the full length protein of human GPIbα. Fragments of GPIb are preferably proteins comprising the ectodomain of GPIb, preferably of human GPIbα, but lacking any transmembrane or intracellular parts of the protein. Most preferably the fragments of GPIb retain a functions to bind to thrombin, or at least comprise a binding site to thrombin, or adjacent protein sequences (10, 20, 50, 100 or 200 amino acids adjacent to such binding site). The variant of GPIb is in some embodiments a protein that is (i) a thrombin binding fragment of GPIb, and/or (ii) a protein having an amino acid sequence which is at least 80% identical to the amino acid sequence shown in SEQ ID NO: 1.

The present invention in the first aspect provides also compounds and methods for the treatment of NAFLD, NASH and/or HCC by interfering with thrombin binding to GPIbα. Such compounds and compositions preferably target GPIbα directly, by, for example, binding specifically to its ectodomain and thereby impairing thrombin binding to GPIbα. In some embodiments however the invention in all of the herein disclosed aspects and embodiments excludes compounds specifically binding to thrombin or α-thrombin.

As used herein, the term "thrombin" refers to the serine protease that converts soluble fibrinogen into insoluble strands of fibrin and catalyses a number of other coagulation-related reactions. This term is not species-specific unless otherwise designated. The term encompasses α-thrombin, which is the native form of thrombin, as well as γ-thrombin, a non-clotting derivative produced from α-thrombin that retains much of its platelet-activating capacity.

The term "ectodomain of GPIb" protein refers to the portion of the GPIb protein that is exposed on the cytoplasmic side of a lipid bilayer of a cell, preferably of a platelet comprising such GPIb protein. Methods for determining the ectodomain of a protein are known in the art (Singer (1990); High et al. (1993), and McVector software, Oxford Molecular). The ectodomain of GPIbα is well known. In the human protein the N-terminal of the protein is extracellular, whereas the C-terminal tail is in the cytoplasm located. The transmembrane region is most likely located between amino acids 532-552 (preferably excluding the signal peptide). Hence, the ectodomain of GPIb in context of the invention preferably comprises a region located in the first 531 consecutive N-terminal amino acids of human GPIbα. Such ectodomain further includes a leucine rich repeat domain, which is a preferred domain targeted in accordance with the herein disclosed invention. Hence, the compound for use in accordance with the invention specifically binds to a leucine rich repeat containing domain in the ectodomain of GPIb. The ectodomain preferably at least comprises at least 100, preferably at least 200 and most preferably at least 290 consecutive amino acids of the N-terminus, and preferably beginning with the N-terminus, of GPIb.

In some embodiments the present invention pertains to a compound for use according to the disclosed medical applications, wherein the compound binds specifically to exosite I and/or II of GPIb.

In particular preferred embodiments of the invention the compound binding to the ectodomain of GPIb competes with thrombin, preferably α-thrombin, for binding to the ectodomain of GPIb. By competitive binding the compound thus impairs the normal interaction between GPIb and thrombin. However, other mechanisms of the compounds of the invention of impairing thrombin-GPIb interaction are also encompassed by the invention.

In another embodiment the compound binding to the ectodomain of GPIb does not compete with von Willebrandt factor (vWF), P-selectin, Mac-1, coagulation factor XI and/or coagulation factor XII for a binding to the ectodomain of GPIb. Hence, the compound of the invention preferably does not affect or impair such GPIb ligands, nor their biological pathways or effects.

The term "non-alcoholic fatty liver disease" or "NAFLD", as used herein, refers to a group of conditions having in common the accumulation of fat in the hepatocytes, NAFLD ranges from simple fatty liver (steatosis), to non-alcoholic steatohepatitis (NASH), to cirrhosis (irreversible, advanced scarring of the liver). The term "NAFLD" includes any stage or degree of progression of the disease.

The term "non-alcoholic steatohepatitis" or "NASH", as used herein, relates to a significant form of chronic liver disease characterized by inflammatory and fatty infiltration of the liver that is not associated with alcohol consumption.

The term "disorder or condition associated with NAFLD or NASH" in context of the invention shall be any disorder of condition which is pathologically directly affected or influenced by NAFLD or NASH. Preferably the term includes a disorder or condition developing from NAFLD or NASH, meaning that such disorder or condition is caused by a progressing, for example untreated, NAFLD or NASH. A preferred example is a liver cirrhosis or hepatocellular carcinoma (HCC).

As used herein the term "hepatocellular carcinoma" or "HCC" refers to the most common type of liver cancer, also called malignant hepatoma. HCC may have many different causes, but in some preferred embodiments of the present invention, the underlying cause of the liver disease is non-alcoholic fatty liver disease (NAFLD). NAFLD is the most common liver disorder in the Western industrialized countries. It is considered to be the hepatic manifestation of the metabolic syndrome. Thus, NAFLD tends to develop in people who are overweight or obese, and/or who have diabetes, high cholesterol or high triglycerides. For most people, NAFLD cause no signs and symptoms, and no complications. But in some people with NAFLD, the fat that accumulates in the liver can cause inflammation and scarring in the liver that is believed to result in fibrosis and cirrhosis. This more serious form of NAFLD is sometimes called non-alcoholic steatohepatitis (NASH). It is worth noting that metabolic syndrome and type 2 diabetes have been demonstrated to be independent risk factors of HCC - patients suffering from such risk factors are preferred patients to be treated in accordance of the invention because preferably the treatment also includes preventive treatments using the compounds and methods of the invention to avoid, or reduce the chance of, developing NAFLD/NASH and/or HCC.

A treatment in context of the invention includes a preventive treatment in order to reduce the chance of developing the disorder, for example in a patient suffering from a risk factor of the disease. Preferably however, the treatment of the invention is alleviation or a reduced progression of NASH and/or HCC, or its symptoms or complications. A subject being at risk of developing NASH, is for example, and preferably a diabetic patient, an obese patient, or a patient suffering from the metabolic syndrome or from another metabolic disorder.

Preferred in some embodiments is that the treatment of the invention is a reduced, stalled, or reversed progression of NAFLD/NASH into liver cirrhosis, preferably a reduced, stalled, or reversed progression of NASH into hepatocellular carcinoma (HCC). Further preferred is that the treatment is a prevention of HCC in a NASH-patient at risk to develop cirrhosis and/or HCC.

In some preferred embodiments of the invention, the subject to be treated does not have a conditions selected from the following group consisting of alcoholic liver injury, drug-induced liver injury, chronic active hepatitis, hepatic steatosis and hepatocyte apoptosis, and preferably wherein the patient suffers from an inflamed fatty liver.

As mentioned above it was surprisingly found in context of the invention that the ectodomain of GPIb is involved in the development or progression of NAFLD and in particular NASH. It is known that GPIb has a wide variety of biological functions and can bind many different ligands. However, the present invention specifically identifies the interaction of thrombin with GPIb as a key target for the pathology of NAFLD/NASH and HCC. Hence, the invention provides means for impairing such interaction as a new therapeutic strategy for the treatment and prevention of the indicated disorders, as is supported by the appended examples.

In some embodiments of the invention the compound binds to a thrombin binding site, preferably to a α-thrombin binding site, wherein said binding site is located within the ectodomain of GPIb; and/or wherein said compound binds the ectodomain of GPIb such that upon binding between the compound and GPIb a further binding of a thrombin (such as α-thrombin) to GPIb is reduced or impaired, for example by sterically hindering a GPIb-thrombin interaction and/or by changing the 3-dimensional conformation of GPIb.

Preferably the compound for use according to the invention is a compound characterized in that it, preferably when bound to an ectodomain of GPIb, reduces trafficking of platelets to the liver, and/or reduces interaction of platelets with Kupffer cells, and/or reduces platelet aggregate size, and/or reduces platelet area, and/or reduces platelet-endothelium coverage.

Furthermore, the compound for use according the invention is characterized in some alternative or additional embodiments in that the compound, preferably when administered to a NAFLD/NASH/HCC patient, has one or more or a combination of the following activities/effects or characteristics :
- reduces serum cholesterol, liver triglycerides, serum ALT and/or AST levels in a subject administered the compound; and/or
- reduces serum LDL- and/or serum HDL-cholesterol in a subject administered the compound; and/or
- prevents dysregulation of mRNA expression of lipid metabolism-related genes in a subject administered the compound; and/or
- attenuates liver damage in a subject administered the compound; and/or
- reduces intra-hepatic CD8+ T- and/or NKT-cells; and/or
- reduces intrahepatic neutrophil accumulation and/or macrophage influx/activation; and/or
- reduces protein expression of several pro-inflammatory and homeostatic cytokines/chemokines; and/or
- reduces lipid accumulation;
or preferably:
- Reduced intrahepatic platelet accumulation, and/or
- Reduced intrahepatic inflammation, and/or reduced intrahepatic immune cell infiltration, and/or
- Reduced steatosis, and/or
- Reduced liver volume, and/or
- Reduced liver triglycerides, and/or
- Reduced liver damage, and/or
- Reduced NAS, and/or
- Dampened liver fibrosis.

A compound specifically binding to an ectodomain of GPIb protein, or of a variant of GPIb protein, is preferably a protein binding compound (PBC), preferably an antigen binding protein (ABP).

The terms "specifically binding an ectodomain of GPIb ", as used herein, refer to binding of the compound to the respective antigen (target) or antigen-expressing cell, measured by ELISA, wherein said ELISA preferably comprises coating the respective antigen (ectodomain of GPIb) to a solid support, adding said compound under conditions to allow the formation of an complex with the respective antigen or protein, detecting said complex by measuring the Optical Density values (OD) using a secondary antibody binding to a compound according to the invention and using a peroxidase-mediated color development, as non-limiting examples.

The term "antigen" according to the invention refers to the antigen used for immunization or a protein comprising said antigen as part of its protein sequence. For example, for immunization a fragment of the extracellular domain of a protein (e.g. the first 20 amino acids) can be used and for detection/assay and the like the extracellular domain of the protein or the full-length protein can be used.

Such a binding compound of the invention is preferably a polypeptide, peptide, glycoprotein, a peptidomimetic, an antigen binding construct (for example, an antibody, antibody-like molecule (e.g. nanobody) or other antigen binding derivative, or an or antigen binding fragment thereof), a nucleic acid such as a DNA or RNA, for example a ribozyme or an RNA or DNA aptamer, including variants or derivatives thereof such as a peptide nucleic acid (PNA), or is a small molecular compound.

Preferably the compound is an antigen binding construct (for example, an antibody, nanobody, scFv, Fab, antibody-like molecule or other antigen binding derivative, or an antigen binding fragment thereof), that binds said ectodomain of GPIb, or an ectodomain of said variant of GPIb.

In particular embodiments, a compound of the invention can be an antibody or an antigen binding fragment or derivative thereof.

As used herein, the term "antibody" may be understood in the broadest sense as any immunoglobulin (Ig) that enables binding to its epitope. An antibody as such is a species of an ABP. Full length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chain of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to cells or factors, including various cells of the immune system (e.g., effector cells) and the first component (C1q) of the classical complement system. Other forms of antibodies include heavy-chain antibodies, being those which consist only of two heavy chains and lack the two light chains usually found in antibodies. Heavy-chain antibodies include the hcIgG (IgG-like) antibodies of camelids such as dromedaries, camels, llamas and alpacas, and the IgNAR antibodies of cartilaginous fishes (for example sharks). And yet other forms of antibodies include single-domain antibodies (sdAb, called Nanobody by Ablynx, the developer) being an antibody fragment consisting of a single monomeric variable antibody domain. Single-domain antibodies are typically produced from heavy-chain antibodies, but may also be derived from conventional antibodies.

Antibodies (or those from which fragments thereof can be isolated) can include, for instance, chimeric, humanized, (fully) human, or hybrid antibodies with dual or multiple antigen or epitope specificities, antibody fragments and antibody sub-fragments, e.g., Fab, Fab' or F(ab')2 fragments, single chain antibodies (scFv) and the like (described below), including hybrid fragments of any immunoglobulin or any natural, synthetic or genetically engineered protein that acts like an antibody by binding to a specific antigen to form a complex.

Accordingly, in certain embodiments a compound of the invention can comprise an antibody heavy chain, or an antigen binding fragment thereof, and/or an antibody light chain, or an antigen binding fragment thereof.

The term "nanobody" (or VHH, Hamers-Casterman et al, 1993) designates a single chain polypeptide consisting essentially of three CDRs (complementarity-determining regions CDR1, CDR2 and CDR3) separated by four FR domains (for Framework regions) and essentially devoid of a light chain or a constant domain. The terms "nanobodies", "nanobody", "VHH", "VHH antibody fragment" or "single-domain antibody" are used interchangeably. Nanobodies typically have the following structure: FR1-CDR1-FR2- CDR2-FR3-CDR3-FR4. Preferably, the nanobodies of the invention are synthetic molecules produced by recombinant or chemical technologies. Nanobodies present high epitope specificity and affinity. They are about ten times smaller than conventional IgG molecules. They are single-chain polypeptides, very stable, resisting extreme pH and temperature conditions. Moreover, they can resist to the action of proteases. Nanobodies as compounds binding specifically to an ectodomain of GPIb are preferred in some embodiments of the invention. Preferably nanobodies in accordance of the invention are obtained from alpacas.

In some embodiments which are preferred the compound of the invention, and preferably wherein the compound is an antibody or nanobody, or antigen binding fragments or derivatives thereof, competes with the known anti-GPIbα antibody pOp/B, as disclosed in Kleinschnitz er al. (circulation vol. 115 p.2323-2330 (2007)). In other preferred embodiments the compound of the invention is the anti-GPIbα antibody pOp/B, or an antigen binding fragment thereof, preferably as defined herein.

In yet some further embodiments which may be preferred the compound of the invention, and preferably wherein the compound is an antibody or nanobody, or antigen binding fragments or derivatives thereof, is not, or does not comprise the known anti-GPIba antibody pOp/B, as disclosed in Kleinschnitz er al. (Circulation vol. 115 p.2323-2330 (2007)), or does not comprise an antigenic binding fragment thereof, such as a variable domain heavy and/or light chain sequence of such antibody.

In **a second aspect,** the invention pertains to a pharmaceutical composition comprising a compound according to the first aspect. Preferably the pharmaceutical composition of the invention is for use according to the first aspect. Furthermore, pharmaceutical compositions of the invention preferably further comprise and a pharmaceutically acceptable carrier and/or excipient.

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions. In certain embodiments, the pharmaceutically acceptable carrier comprises serum albumin.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical) and transmucosal administration. Most preferably the route of administration is a route that directly targets the liver of a subject to be treated.

In therapeutic applications, compositions are administered to a patient already suffering from a NAFLD, NASH or HCC, as described, in an amount sufficient to cure or at least partially stop the symptoms of the disease and its complications. An appropriate dosage of the pharmaceutical composition is readily determined according to any one of several well-established protocols. For example, animal studies (for example on mice or rats) are commonly used to determine the maximal tolerable dose of the bioactive agent per kilogram of weight. In general, at least one of the animal species tested is mammalian. The results from the animal studies can be extrapolated to determine doses for use in other species, such as humans for example. What constitutes an effective dose also depends on the nature and severity of the disease or condition, and on the general state of the patient's health.

In prophylactic applications, compositions containing, for example compound specifically binding to an ectodomain of GPIb, are administered to a patient susceptible to or otherwise at risk of a hepatic disease. Such an amount is defined to be a "prophylactically effective" amount or dose. In this use, the precise amount depends on the patient's state of health and weight.

In both therapeutic and prophylactic treatments, the antagonist contained in the pharmaceutical composition can be administered in several dosages or as a single dose until a desired response has been achieved. The treatment is typically monitored and repeated dosages can be administered as necessary. Compounds of the invention may be administered according to dosage regimens established whenever impairment of thrombin interaction with GPIb is required.

The daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 10 mg/kg of body weight per day. It will be understood, however, that the specific dose level and frequency of dosage for any particular patient may be varied and will depend upon a variety of factors including the activity of the specific compound employed, the metabolic stability, and length of action of that compound, the age, the body weight, general health, sex, diet, mode and time of administration, rate of excretion, drug combination, the severity of the particular condition, and the host undergoing therapy.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

The appropriate unit forms of administration include forms for oral administration, such as tablets, gelatine capsules, powders, granules and solutions or suspensions to be taken orally, forms for sublingual and buccal administration, aerosols, implants, forms for subcutaneous, intramuscular, intravenous, intranasal or intraocular administration and forms for rectal administration.

In the pharmaceutical compositions of the present invention, the active principle is generally formulated as dosage units containing from 0.5 to 1000 mg, preferably from 1 to 500 mg, more preferably from 2 to 200 mg of said active principle per dosage unit for daily administrations.

When preparing a solid composition in the form of tablets, a wetting agent such as sodium laurylsulfate can be added to the active principle optionally micronized, which is then mixed with a pharmaceutical vehicle such as silica, gelatine, starch, lactose, magnesium stearate, talc, gum arabic or the like. The tablets can be coated with sucrose, with various polymers or other appropriate substances or else they can be treated so as to have a prolonged or delayed activity and so as to release a predetermined amount of active principle continuously.

A preparation in the form of gelatin capsules is obtained by mixing the active principle with a diluent such as a glycol or a glycerol ester and pouring the mixture obtained into soft or hard gelatine capsules.

A preparation in the form of a syrup or elixir can contain the active principle together with a sweetener, which is preferably calorie-free, methyl-paraben and propylparaben as an antiseptic, a flavoring and an appropriate color.

The water-dispersible powders or granules can contain the active principle mixed with dispersants or wetting agents, or suspending agents such as polyvinylpyrrolidone, and also with sweeteners or taste correctors.

Rectal administration is effected using suppositories prepared with binders which melt at the rectal temperature, for example cacao butter or polyethylene glycols.

Parenteral, intranasal or intraocular administration is effected using aqueous suspensions, isotonic saline solutions or sterile and injectable solutions which contain pharmacologically compatible dispersants and/or wetting agents, for example propylene glycol, butylene glycol, or polyethylene glycol.

Thus a co-solvent, for example an alcohol such as ethanol or a glycol such as polyethylene glycol or propylene glycol, and a hydrophilic surfactant such as Tween. RTM80, can be used to prepare an aqueous solution injectable by intravenous route. The active principle can be solubilized by a triglyceride or a glycerol ester to prepare an oily solution injectable by intramuscular route.

Transdermal administration is effected using multilaminated patches or reservoirs into which the active principle is in the form of an alcoholic solution.

Administration by inhalation is effected using an aerosol containing for example sorbitan trioleate or oleic acid together with trichlorofluoromethane, dichlorotetrafluoroethane or any other biologically compatible propellant gas.

The active principle can also be formulated as microcapsules or microspheres, optionally with one or more carriers or additives.

Among the prolonged-release forms which are useful in the case of chronic treatments, implants can be used. These can be prepared in the form of an oily suspension or in the form of a suspension of microspheres in an isotonic medium.

In **a third aspect,** the invention pertains to a method for producing an antibody, antibody-like molecule, or antigenic binding fragment thereof, comprising the steps of: immunizing a non-human animal with a peptide, the peptide comprising, or consisting of, a sequence of the ectodomain of GPIb, and isolating from that immunized mammal an immune cell expressing such antibody, antibody-like molecule or antigenic binding fragment thereof.

Preferably the GPIb and/or ectodomain thereof is as defined herein above for the first aspect of the invention.

Accordingly, in a particular aspect, the invention also relates to a method for (or of) identifying, generating and/or producing an ABP that (eg, specifically) binds to an ectodomain of GPIb, or a variant or fragment/epitope thereof, the method comprising the use of such domain (or variant or fragment/epitope): (i) to screen a display library of a plurality of ABPs (eg a phage display library); or (ii) to immunise an animal, in particular a mammal (such as a mouse, rat, rabbit, goat, camel or llama).

In particular of such embodiments, a display library (eg, a phage display library) is screened that displays a plurality of ABPs, where, preferably, such library is screened for ABPs that bind such protein.

Immunising an animal, in preferred embodiments, comprises a step of administering to the animal an immunisation composition comprising such ectodomain of GPIb or variant thereof or at least one or more epitopes thereof or comprised therein (eg, either as a protein or as a nucleic acid encoding such domain or epitope thereof), and optionally together with a pharmaceutically acceptable carrier and/or excipient, more preferably such immunisation composition comprises one or more adjuvants. An immunising composition in accordance with the invention elicits an immune response in the immunised animal which is specific for the ectodomain of GPIb (or variant thereof), preferably by generation of antibodies against such protein. Following immunisation, certain such embodiments of the invention can include a further step of isolating from the animal: (i) sera that comprises an ABP that specifically binds to said ectodomain of GPIb (or variant thereof); and/or (ii) B cells that express an ABP that specifically binds to said domain of IGSF11 (or variant thereof).

In any of such method or use aspects, an ABP for use in medicine is, typically (and eg as described in further detail elsewhere herein):

Alternatively, or in addition, in any of such method or use aspects, the ABP (and eg as described in further detail elsewhere herein):
- reduces serum cholesterol, liver triglycerides, serum ALT and/or AST levels in a subject administered the compound; and/or
- reduces serum LDL- and/or serum HDL-cholesterol in a subject administered the compound; and/or
- prevents dysregulation of mRNA expression of lipid metabolism-related genes in a subject administered the compound; and/or
- attenuates liver damage in a subject administered the compound; and/or
- reduces intra-hepatic CD8+ T- and/or NKT-cells; and/or
- reduces intrahepatic neutrophil accumulation and/or macrophage influx/activation; and/or
- reduces protein expression of several pro-inflammatory and homeostatic cytokines/chemokines; and/or
- reduces lipid accumulation;

In any of such aspects (and eg as described in further detail elsewhere herein), the ABP can be an antibody or nanobody, or an antigen binding fragment thereof. In particular, the antibody/nanobody can be a monoclonal antibody, or wherein the antigen binding fragment can be a fragment of a monoclonal antibody. For example, such an antibody can be a human antibody a humanised antibody or a chimeric-human antibody, or the antigen binding fragment can be a fragment of a human antibody a humanised antibody or a chimeric-human antibody.

In **a fourth aspect,** the invention pertains a method for identifying a compound suitable for the treatment of non-alcoholic fatty liver disease (NAFLD) or non-alcoholic steatohepatitis (NASH), or of a disorder or condition associated with NAFLD or NASH, such as a disorder or condition developing from NAFLD or NASH, the method comprising the steps of
**(a)** Providing (x) a first cell expressing a protein or mRNA of GPIb, or of a variant of GPIb, or providing (y) a first test protein comprising, or (essentially) consisting of, an ectodomain of GPIb, or of a variant of GPIb,
**(b)** Providing a candidate compound,
**(c)** Bringing into contact the first cell or first test protein and the candidate compound, and
**(d)** Determining subsequent to step (c), either or both of:
   **(i)** A binding of the candidate compound to the first cell or to the first test protein; and/or
   **(ii)** A binding of the candidate compound to first cell or first test protein which binding is competitive with a thrombin protein;
wherein a binding in (i) or a competitive binding in (ii) indicates the candidate compound as suitable for the treatment of non-alcoholic fatty liver disease (NAFLD) or non-alcoholic steatohepatitis (NASH), or of a disorder or condition associated with NAFLD or NASH, such as a disorder or condition developing from NAFLD or NASH.

In a preferred embodiment the first cell is a platelet.

In some embodiments the binding of the candidate compound to the first cell or first test protein is an interaction between the candidate compound and the ectodomain of GPIb, preferably is a binding to a thrombin binding site, preferably to a α-thrombin binding site, wherein said binding site is located within the ectodomain of GPIb; and/or wherein said candidate compound binds the ectodomain of GPIb such that upon binding between the compound and GPIb a further binding of a thrombin (such as α-thrombin) to GPIb is reduced or impaired, for example by sterically hindering a GPIb-thrombin interaction and/or by changing the 3-dimensional conformation of GPIb.

In **a fifth aspect,** the invention pertains to a method for the treatment of non-alcoholic fatty liver disease (NAFLD) or non-alcoholic steatohepatitis (NASH), or of a disorder or condition associated with NAFLD or NASH, such as a disorder or condition developing from NAFLD or NASH, wherein the treatment comprises a step of administering to a subject in need of the treatment a therapeutically effective amount of a compound which specifically binds to an ectodomain of platelet glycoprotein Ib (GPIb). Preferably wherein the compound is as disclosed herein above.

In view of the above, it will be appreciated that the present invention also relates to the following itemised embodiments:
**Item 1: A compound for use in the treatment of non-alcoholic fatty liver disease (NAFLD) or non-alcoholic steatohepatitis (NASH),** or of a disorder or condition associated with NAFLD or NASH, such as a disorder or condition developing from NAFLD or NASH (such as Hepatocellular Carcinoma), wherein the compound specifically binds to an ectodomain of platelet glycoprotein Ib (GPIb).
**Item 2:** The compound for use according to item 1, wherein the compound binds to a thrombin binding site, preferably to a α-thrombin binding site, wherein said binding site is located within the ectodomain of GPIb; and/or wherein said compound binds the ectodomain of GPIb such that upon binding between the compound and GPIb a further binding of a thrombin (such as α-thrombin) to GPIb is reduced or impaired, for example by sterically hindering a GPIb-thrombin interaction and/or by changing the 3-dimensional conformation of GPIb.
**Item 3:** The compound for use according to item 1 or 2, wherein the compound reduces trafficking of platelets to the liver, and/or reduces interaction of platelets with Kupffer cells, and/or reduces platelet aggregate size, and/or reduces platelet area, and/or reduces platelet-endothelium coverage.
**Item 4:** The compound for use according to any one of items 1 to 3, wherein the compound
   **(a)** reduces serum cholesterol, liver triglycerides, serum ALT and/or AST levels in a subject administered the compound; and/or
   **(b)** reduces serum LDL- and/or serum HDL-cholesterol in a subject administered the compound; and/or
   **(c)** prevents dysregulation of mRNA expression of lipid metabolism-related genes in a subject administered the compound; and/or
   **(d)** attenuates liver damage in a subject administered the compound; and/or
   **(e)** reduces intra-hepatic CD8+ T- and/or NKT-cells; and/or
   **(f)** reduces intrahepatic neutrophil accumulation and/or macrophage influx/activation; and/or
   **(g)** reduces protein expression of several pro-inflammatory and homeostatic cytokines/chemokines; and/or
   **(h)** reduces lipid accumulation.
**Item 5:** The compound for use according to any one of items 1 or 4, wherein the compound specifically binds to a leucine rich repeat containing domain in the ectodomain of GPIb.
**Item 6:** The compound for use according to any one of items 1 to 5, wherein GPIb is GPIb α-chain (GPIbα).
**Item 7:** The compound for use according to any one of items 1 to 6, wherein GPIb is human GPIb, or a variant thereof, and preferably wherein human GPIb comprises an amino acid sequence shown in SEQ ID NO: 1 (UniProt ref: P07359).
**Item 8:** The compound for use according to item 7, wherein the variant of GPIb is (i) a thrombin binding fragment of GPIb, and/or (ii) a protein having an amino acid sequence which is at least 80% identical to the amino acid sequence shown in SEQ ID NO: 1.
**Item 9:** The compound for use according to any one of items 1 to 8, wherein the compound binds specifically to exosite I and/or II of GPIb.
**Item 10:** The compound for use according to any one of items 1 to 9, wherein the ectodomain at least comprises at least 100, preferably at least 200 and most preferably at least 290 consecutive amino acids of the N-terminus, and preferably beginning with the N-terminus, of GPIb.
**Item 11:** The compound for use according to any one of items 1 to 10, wherein the compound competes with thrombin, preferably α-thrombin, for binding to the ectodomain of GPIb.
**Item 12:** The compound for use according to any one of items 1 to 11, wherein the compound does not compete with von Willebrandt factor (vWF), P-selectin, Mac-1, coagulation factor XI and/or coagulation factor XII.
**Item 13:** The compound for use according to any one of items 1 to 12, wherein the compound when administered to a NAFLD/NASH/HCC patient has any one of, or a combination of, the following effects:
   **(a)** Reduced intrahepatic platelet accumulation,
   **(b)** Reduced intrahepatic inflammation, and/or reduced intrahepatic immune cell infiltration,
   **(c)** Reduced steatosis,
   **(d)** Reduced liver volume,
   **(e)** Reduced liver triglycerides,
   **(f)** Reduced liver damage,
   **(g)** Reduced NAS, and/or
   **(h)** Dampened liver fibrosis.
**Item 14:** The compound for use according to any one of items 1 to 13, wherein the compound competes with pop/B-antibody, or with an antigen binding fragment thereof, for binding to GPIb.
**Item 15:** The compound for use according to any one of items 1 to 14, wherein the compound is selected from a protein binding compound (PBC), preferably an antigen binding protein (ABP), and may be a polypeptide, peptide, glycoprotein, a peptidomimetic, an antigen binding construct (for example, an antibody, antibody-like molecule (e.g. nanobody) or other antigen binding derivative, or an or antigen binding fragment thereof), a nucleic acid such as a DNA or RNA, for example a ribozyme or an RNA or DNA aptamer, including variants or derivatives thereof such as a peptide nucleic acid (PNA), or is a small molecular compound.
**Item 16:** The compound for use according to any one of items 1 to 15, wherein the compound is an antigen binding construct (for example, an antibody, nanobody, scFv, Fab, antibody-like molecule or other antigen binding derivative, or an antigen binding fragment thereof), that binds said ectodomain of GPIb, or an ectodomain of said variant of GPIb.
**Item 17:** The inhibitor for use according to any of items 1 to 16, wherein the treatment is a alleviation or a reduced progression of NASH and/or HCC.
**Item 18:** The compound for use according to any one of items 1 to 17, wherein the treatment is a reduced, stalled, or reversed progression of NAFLD/NASH into liver cirrhosis, preferably a reduced, stalled, or reversed progression of NASH into hepatocellular carcinoma (HCC).
**Item 19:** The compound for use according to any one of items 1 to 18, wherein the treatment is a prevention of HCC in a NASH-patient at risk to develop cirrhosis and/or HCC.
**Item 20:** The compound for use according to any one of items 1 to 19, wherein the treatment is performed in a patient at risk of developing NASH, such as a diabetic patient, an obese patient, or a patient suffering from the metabolic syndrome or from another metabolic disorder.
**Item 21:** The compound for use according to any one of items 1 to 20, wherein the subject to be treated does not have a conditions selected from the following group consisting of alcoholic liver injury, drug-induced liver injury, chronic active hepatitis, hepatic steatosis and hepatocyte apoptosis, and preferably wherein the patient suffers from an inflamed fatty liver.
**Item 22:** The compound for use according to any one of items 1 to 21, wherein the disorder or condition associated with NAFLD or NASH is a tumour disease, such as hepatocellular cancer (HCC).
**Item 23: A pharmaceutical composition** comprising the compound recited in any one of items 1 to 22.
**Item 24:** The pharmaceutical composition according to item 23, for use recited in any one of item 1 to 20.
**Item 25: A method for producing an antibody, or antigenic binding fragment thereof,** comprising the steps of: immunizing a non-human animal with a peptide comprising, or consisting of, the ectodomain of GPIb, and isolating from that immunized mammal an immune cell expressing such antibody.
**Item 26:** The method according to item 23, wherein such antibody is a compound recited in any one of items 1 to 22.
**Item 27: A method for identifying a compound suitable for the treatment of non-alcoholic fatty liver disease (NAFLD) or non-alcoholic steatohepatitis (NASH),** or of a disorder or condition associated with NAFLD or NASH, such as a disorder or condition developing from NAFLD or NASH, the method comprising the steps of
   **(a)** Providing (x) a first cell expressing a protein or mRNA of GPIb, or of a variant of GPIb, or providing (y) a first test protein comprising, or (essentially) consisting of, an ectodomain of GPIb, or of a variant of GPIb,
   **(b)** Providing a candidate compound,
   **(c)** Bringing into contact the first cell or first test protein and the candidate compound, and
   **(d)** Determining subsequent to step (c), either or both of:
      **(i)** A binding of the candidate compound to the first cell or to the first test protein; and/or
      **(ii)** A binding of the candidate compound to first cell or first test protein which binding is competitive with a thrombin protein;
   wherein a binding in (i) or a competitive binding in (ii) indicates the candidate compound as suitable for the treatment of non-alcoholic fatty liver disease (NAFLD) or non-alcoholic steatohepatitis (NASH), or of a disorder or condition associated with NAFLD or NASH, such as a disorder or condition developing from NAFLD or NASH.
**Item 28:** The method according to item 27, wherein the first cell is a platelet.
**Item 29:** The method according to item 27 or 28, wherein the binding of the candidate compound to the first cell or first test protein is an interaction between the candidate compound and the ectodomain of GPIb, preferably is a binding to a thrombin binding site, preferably to a α-thrombin binding site, wherein said binding site is located within the ectodomain of GPIb; and/or wherein said candidate compound binds the ectodomain of GPIb such that upon binding between the compound and GPIb a further binding of a thrombin (such as α-thrombin) to GPIb is reduced or impaired, for example by sterically hindering a GPIb-thrombin interaction and/or by changing the 3-dimensional conformation of GPIb.
**Item 30: A method for the treatment of non-alcoholic fatty liver disease (NAFLD) or non-alcoholic steatohepatitis (NASH),** or of a disorder or condition associated with NAFLD or NASH, such as a disorder or condition developing from NAFLD or NASH, wherein the treatment comprises a step of administering to a subject in need of the treatment a therapeutically effective amount of a compound which specifically binds to an ectodomain of platelet glycoprotein Ib (GPIb).
**Item 31:** The method according to item 30, wherein the compound is a compound recited in any one of items 1 to 22.

### BRIEF DESCRIPTION OF THE FIGURES AND SEQUENCES

The figures show:
**Figure 1****:** shows the natural history of NASH and HCC incidence and schematic representation of the sequential pathophysiological states of non-alcoholic fatty liver disease and liver cancer. Sedentary lifestyle and high caloric intake set the ground for steatosis and non-alcoholic steatohepatitis (NASH). Steatosis and NASH appear to be highly dynamic and most likely reversible, in contrast to NASH induced HCC. NASH can cause liver cancer in the presence or absence of liver fibrosis and liver cirrhosis. NASH can cause liver cancer in the absence of fibrosis/ cirrhosis in approximately 30% of all cases.

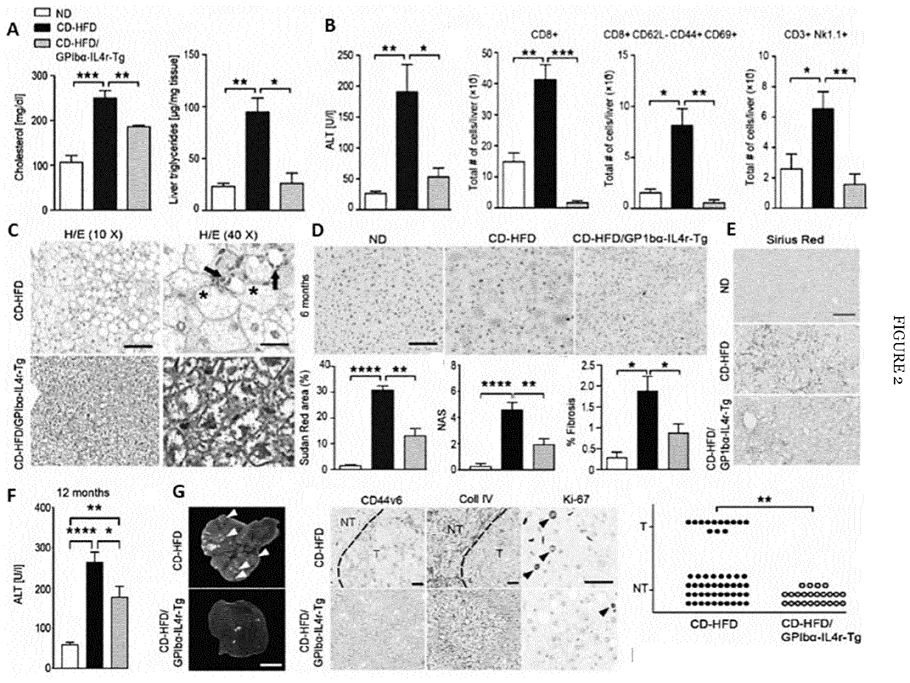

**Figure 2****:** shows platelet derived GPIbα inhibition is a feasible target for NASH. **(A)** Serum cholesterol, liver triglycerides and **(B)** ALT levels of 6 months ND, CD-HFD or CD-HFD/*hIL4rα*/*GP1bα-Tg* fed mice (n≥ 4/group). Quantification by flow cytometry of intrahepatic immune cells **((left)** CD8+ T-cells, **(middle)** activated CD8+ T-cells, **(right)** NKT-cells) of mice shown in (i) (n≥ 4/group). **(C)** Representative H/E staining of mice shown, indications of damaged hepatocytes (asterisks) and satellitosis (arrows), scale bars: 100 µm in 10X and 25 µm in 40X. **(D)** Sudan red staining and quantification of Sudan red-positive areas, NAS evaluation, **(E)** fibrosis quantification and Sirius red staining of 6 months ND, CD-HFD or CD-HFD/*hIL4rα*/*GP1bα-Tg* fed mice (n≥ 4/group). **(F)** ALT levels of 12 months ND, CD-HFD or CD-HFD/*hIL4rα*/*GP1bα-Tg* fed mice (n≥ 10/group). **(G)** Macroscopical images of tumors of mice shown in (r), tumor nodules are indicated by arrowhead), scale bar: 750 mm. HCC characterization by CD44v6, Collagen IV (Coll IV) and Ki67 staining form mice shown. Arrowheads indicate positive hepatocytes, dashed line indicates tumor (T) border, scale bar: 200 µm (CD44v6 and Coll IV), 50 µm (Ki67). HCC incidence (T=HCC; NT= non-tumor) from 12 months CD-HFD or CD-HFD/*hIL4rα*/*GP1bα-Tg* fed mice, (CD-HFD: n=13/51; CD-HFD/*hIL4rα*/*GP1bα-Tg* n=0/24). All data are shown as mean ± SEM.*: P < 0.05. **: P < 0.01. ***: P < 0.001. ****: P < 0.0001. N.s.: Not significant. Adapted from Mahlemir et al., Nature Medicine 2019, incorporated herein by reference in its entirety.

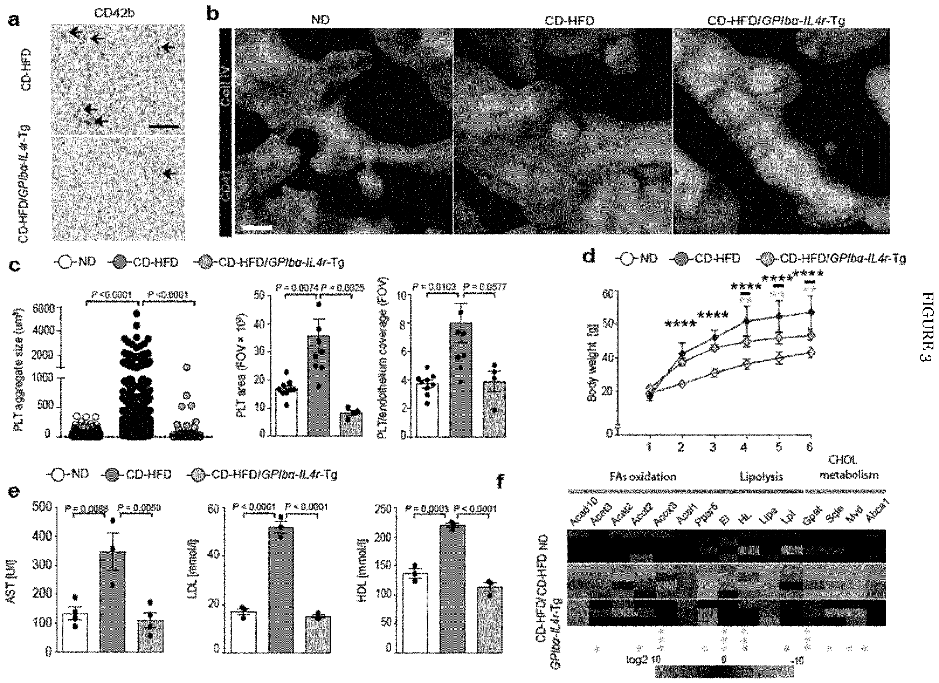

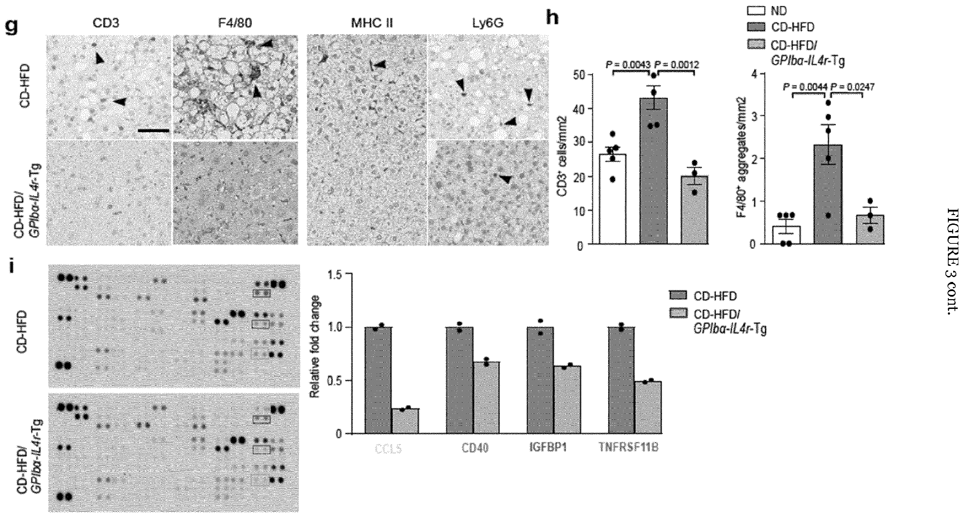

**Figure 3****:** shows that a lack of functional platelet GPIba prevents liver steatosis, injury and inflammation **(a)** Representative CD42b staining of 6 months CD-HFD or CD-HFD/hIL4rα/GPIbα-Tg fed mice (ND n=5 mice, CD-HFD=5 mice, CD-HFD/hIL4rα/GPIbα-Tg n=3 mice) scale bar: 50 µm. **(b)** 3D confocal images platelet (green)/liver endothelium (grey) interaction (ND n=5 mice, CD-HFD=5 mice, CDHFD/hIL4rα/GPIbα-Tg n=4 mice) scale bar: 20 µm. **(c)** Quantification of platelet (PLT) aggregate size, overall PLT surface area and quantification of platelet/liver endothelium coverage in focus of view (FOV; ND n=9 mice, CD-HFD=9 mice, CD-HFD/hIL4rα/GPIbα-Tg n=4 mice). For visualization of intravascular events, the transparency of the sinusoidal rendering was set to 50%. **(d)** Body weight development (ND n=5 mice, CD-HFD=4 mice, CD-HFD/hIL4rα/GPIbα-Tg n=3 mice). Statistics: ND vs CD-HFD (black asterisks), CD-HFD vs CD-HFD/hIL4rα/GPIbα-Tg (yellow asterisks). **(e)** AST (ND n=4 mice, CD-HFD=3 mice, CD-HFD/hIL4rα/GPIbα-Tg n=4 mice), LDL and HDL levels (n=3 mice/group) **(f)** Real-time qPCR analysis for genes involved in lipid metabolism/β-oxidation (ND n=4 mice, CDHFD= 4 mice, CD-HFD/hIL4rα/GPIbα-Tg n=3 mice). Statistics: CD-HFD vs CD-HFD/hIL4rα/GPIbα-Tg mice (yellow asterisks). **(g)** Representative CD3, F4/80, MHCII and Ly-6G stains (ND n=5 mice, CDHFD= 5 mice, CD-HFD/hIL4rα/GPIbα-Tg n=3 mice), scale bar: 50µm **(h)** Quantification of CD3+ cells and F4/80+ aggregates (ND n=5 mice, CD-HFD=5 mice, CD-HFD/hIL4rα/GPIbα-Tg n=3 mice). **(i)** Analysis of intrahepatic cytokine/chemokine profile of pooled liver tissues of mice shown in (a) (n=2/group). All data are shown as mean ± SEM. Data in (c), (e) and (h) were analysed by one-way analysis of variance with the post hoc Tukey multiple comparison test. Data in (d) were analysed by two-way analysis of variance with the post hoc Bonferroni multiple comparison test. Data in (f) were analyzed by two-tailed Mann Whitney's test. N.s.: not significant *: P < 0.05. **: P < 0.01. ***: P < 0.001. ****: P < 0.0001.

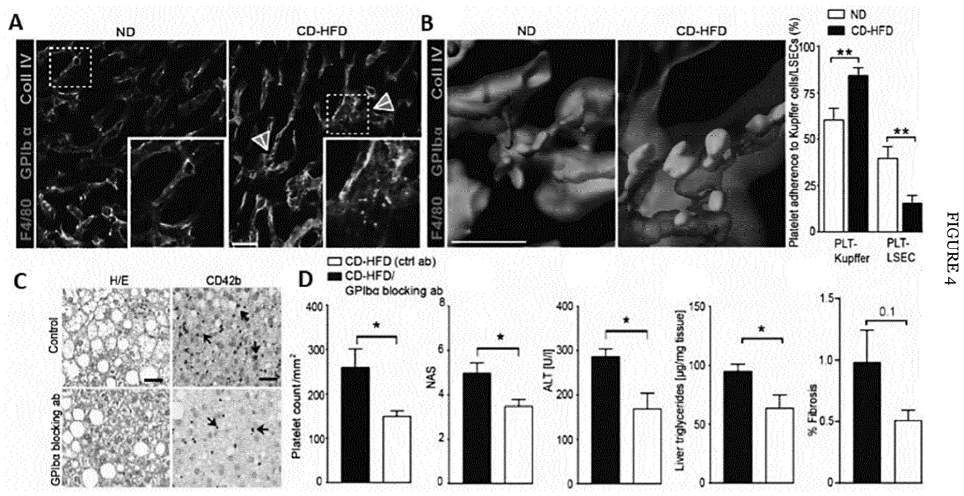

**Figure 4****:** shows that anti-GPIba antibody treatment reduces NASH and fibrosis. **(A)** Representative 3D confocal images of GPIbα (green, green arrowheads)/Kupffer cells (red, red arrowheads) interaction of 6 months ND or CD-HFD fed mice. Liver endothelium (grey), scale bar: 30 µm **(B)** High magnification 3D confocal images and quantification of GPIbα (green)/Kupffer cells (red) and GPIbα (green)/LSECs (grey) interaction in 6 months ND or CD-HFD fed mice (n= 4/group), scale bar: 3 µm. For visualization of intravascular events, the transparency of the sinusoidal rendering was set to 50%. **(C)** Representative H/E and CD42b staining after 5 weeks of GPIbα blocking or control Fab in 6 months CD-HFD fed mice, scale bar: 50 µm. Platelets are indicated by arrows. **(D)** Platelet quantification, NAS evaluation, ALT levels, liver triglycerides and Sirius red-positive areas quantification of mice shown in (c) (n≥ 5/group).
**Figure 5****:** shows GPIba structures. **(A)** Crystal structure of human GPIba (left) with α-thrombin (right) at 2.6Å. (*http:*//*www.ebi.ac.uk*/)*.* **(B, inset of A)** Crystal structure of the GPIb ectodomain at 1.26Å (McEwan et al., 2010).
**Figure 6****:** shows the immunization of alpcas to generate nanobodies. Alpcas are challenged with the respective antigens (e.g. *in vitro translated*) in combination with adjuvant. After isolation of B-cells - the B-cell deried m-RNA of the VHHS PCR products specific for the variable regions are subcloned into a VHHS phage library and expressed bacterially. Upon first selection of nanobodies the forme rare tested by ELISA and other specific assays - (see also Phase II).

The sequences show:

SEQ ID NO: 1: human platelet glycoprotein Ib alpha chain:
MPLLLLLLLLPSPLHPHPICEVSKVASHLEVNCDKRNLTALPPDLPKDTTILHLSENLLY
TFSLATLMPYTRLTQLNLDRCELTKLQVDGTLPVLGTLDLSHNQLQSLPLLGQTLPALT
VLDVSFNRLTSLPLGALRGLGELQELYLKGNELKTLPPGLLTPTPKLEKLSLANNNLTEL
PAGLLNGLENLDTLLLQENSLYTIPKGFFGSHLLPFAFLHGNPWLCNCEILYFRRWLQD
NAENVYVWKQGVDVKAMTSNVASVQCDNSDKFPVYKYPGKGCPTLGDEGDTDLYDYY
PEEDTEGDKVRATRTVVKFPTKAHTTPWGLFYSWSTASLDSQMPSSLHPTQESTKEQT
TFPPRWTPNFTLHMESITFSKTPKSTTEPTPSPTTSEPVPEPAPNMTTLEPTPSPTTPEPT
SEPAPSPTTPEPTSEPAPSPTTPEPTSEPAPSPTTPEPTPIPTIATSPTILVSATSLITPKSTFL
TTTKPVSLLESTKKTIPELDQPPKLRGVLQGHLESSRNDPFLHPDFCCLLPLGFYVLGLF
WLLFASVVLILLLSWVGHVKPQALDSGQGAALTTATQTTHLELQRGRQVTVPRAWLLF
LRGSLPTFRSSLFLWVRPNGRVGPLVAGRRPSALSQGRGQDLLSTVSIRYSGHSLSL

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Platelet derived GPIba inhibition is a feasible target for NASH

Activation of platelets through GPIbα is a precondition for platelet-derived secretion of cargo-vesicles that contribute to immune cell attraction. To investigate in a genetic model, whether block of a the ectodomain function of GPIbα is important for disease progression, the inventors fed transgenic mice expressing an IL-4rα/GPIbα fusion-protein in a GPIbα^{-/-} background in which the ligand-binding ectodomain of GPIbα is replaced by the α-subunit of the human IL-4 receptor (hIL4rα/GP1bα-Tg) with a CD-HFD for 6 months (Fig. 2). Remarkably, platelet aggregate size, platelet area and platelet-liver endothelium coverage were significantly lower in CDHFD-fed hIL4Rα/GPIbα-Tg mice compared to CD-HFD-fed C57Bl/6 controls (Fig. 3a-c). Both hIL4rα/GPIbα-Tg and C57Bl/6 mice gained weight similarly when fed a CD-HFD (Fig. 3d). Serum cholesterol, liver triglycerides, serum ALT and AST levels were significantly lower in CD-HFD/hIL4rα/GPIbα-Tg mice (Fig. 3e), accompanied by less LDL and HDL cholesterol (Fig. 3e). Similarly, dysregulated mRNA expression of lipid metabolism-related genes in livers of CD-HFD-fed C57BL/6 mice was prevented in livers of CD-HFD/hIL4rα/GPIbα-Tg mice (Fig. 3f). The inventors also observed strong and significant reductions in intrahepatic CD8+ T and NKT cells by flow cytometry analysis. Reduced CD3+ and reduced macrophage influx and activation were observed using immunohistochemistry (Fig. 3g,h)

Interestingly, mice lacking the major platelet adhesion receptors P-selectin (Selp-/-), von-Willebrand-factor (vWF-/-) or Mac-1 (Mac1-/-), the so far described a cognate ligands of the GPIba ectodomain, displayed full blown NASH in the context of a CD-HFD (6 months) (Extended Data Figs. 8-10 of Nature Medicine VOL 25 / APRIL 2019 / 641-655, incorporated herein by reference).

These data indicated that another ligand binding to the ectodomain of GPIbα might be important for GPIbα activation to drive platelet-related immunological, pro-inflammatory function without affecting hemostasis.

Finally, it was investigated whether hIL4rα/GPIbα-Tg mice would develop HCC upon a long term CD-HFD. Of note, hIL4rα/GPIbα-Tg mice receiving CD-HFD for 12 months displayed significantly lower fibrosis, serum ALT levels, and lacked any macro- or microscopical evidence of HCC (Fig. 2F, G). These data, demonstrate that GPIbα is a potent target for a NASH, NASH-HCC therapy.

### Example 2: Platelet derived GPIbα inhibition is a feasible target for NASH

It was hypothesized that GPIbα might indeed mediate platelet-trafficking/activation in inflamed livers during NASH, contributing to efficient immune-cell recruitment to the liver. The inventors thus analyzed the interaction of GPIbα with parenchymal and non-parenchymal liver cells (LSECs; Kupffer cells etc.) in NASH (Fig 4A, 4B). 3D reconstruction revealed most frequent interactions between GPIbα+ platelets and Kupffer cells but less so with LSECs in mouse and in human samples.

To block the major ligand binding domain of GPIbα in 6-months CD-HFD-fed mice Fab fragments of the anti-GPIbα antibody, pop/B was used for 5 weeks. This antibody was demonstrated to bind the ectodomain of GPIbα.

Notably, already this relatively short treatment time-frame significantly reduced intrahepatic platelet accumulation in the presence of a NASH diet. Consequently, steatosis, NAS, liver damage and intrahepatic immune-cell infiltration were significantly reduced (in average by more than 50%); fibrosis was dampened (fig. 4C and 4D). In addition, anti-GPIbα antibody treatment reduced protein expression of several pro-inflammatory and homeostatic cytokines/chemokines - linking intrahepatic platelet activation and subsequent cargo release with mediators of inflammation (Malehmir et al., 2019, Nature Medicine).

### Example 3: Generation of anti- GPIbα nanobodies

Production of nanobodies in alpacas and other camelids has a distinct advantage over other methods in that a much greater diversity in the nanobody pool is achieved offering a better chance of obtaining a nanobody with the desired properties, i.e high affinity, unique epitope, optimal kinetics. The generation of the nanobodies of the invention is depicted in fig 6.

As is understood such nanobodies are prepared with three different antigens to generate nanobodies. One epitope will encompass the whole ectodomain of human GPIbα. The second antigens will contain 30 amino acids 5' and 3' upstream of the human GPIbα ectodomain (see fig 5). The last peptide used for immunization will be a 19 amino acid stretch that encompasses the thrombin binding site located in the human GPIbα ectodomain. Quality control of the respective plasmids for in vitro translation has been already accomplished.

### Example 4: Screening and validation of novel nanobodies

After obtaining a number of antibodies/nanobodies with unknown binding affinity and binding specificity to GPIbα, the produced nanobodies are characterized regarding several biological characteristics: (I) binding to platelets, (II) functional blockade of the thrombin binding domain of GPIbα *in vitro,* (III) functional impact on platelet activation *in vitro.*

Five different assays will be applied:
**(I):** To test the affinity and avidity of the nanobodies the inventors perform BiaCore analysis to test which antibodies have the highest affinity and avidity to human recombinant GPIbα bound to a gold surface.
**(II):** ELISAs will be performed to screen/corroborate the binding of nanobodies to human GPIbα.
(III): A FACS analysis will be performed with fluorophore labelled nanobodies for the binding to platelets.
**(IV):** Thrombin activated platelets will be treated with or without nanobodies and platelet activation (e.g. through recombinant thrombin) will be tested.
**(V)** To reassure the specificity of the binding to thrombin, human GPIbα is expressed on platelets in mice either with the full length of human GPIbα or a mutated version of GPIbα which has one amino acid subsitition that abrogates thrombin binding to GPIbα. Platelets of these mice will be used to test the specificity of the nanobodies *in vivo* and *ex vivo.* Further a block of thrombin binding to GPIba is observed and it is tested whether the anobodies inhibit thombin induced platelet activation (see also IV).

The gained quantitative results will be used to rank the different nanobodies against the thrombin binding domain of GPIbα.

### Example 5: Validation of selected nanobodies in vivo

For in vivo validation several murine models on NASH diet (choline-deficient high fat diet; CD-HFD and Western diet (WD)) are used. Mice will be fed for 6 months with the respective NASH diets and monitored non-invasively with serum markers and ultra sound (US). At the time point at which all mice display NASH (see also Mahlemir et al., Nature Medicine 2019) one will start the treatment with nanobodies for 12 weeks. After 4 and 8 weeks the efficacy of the treatment is tested via serum markers (ALT, AST, Alkaline phosphatase) non-invasively and will closely monitor the behavior of mice - due to the unexpected toxicity of the individual nanobodies. Moreover, platelets are activated *in vivo* and ex vivo to test whether also in the context of non-NASH, non-metabolic syndrome conditions the obtained nanobodies can suppress platelet activation.

This will include C57B/6/J mice which are induced with NASH (CD-HFD and WD) that will be treated with a murine antibody (anti- GPIba antibody) - to have a base line comparison and evaluate the reversibility of NASH - based on the publication by Mahlemir et al., 2019 Nature Medicine (group of mice n=8).
- NASH CD-HFD 6 months diet - followed by 12 weeks treatment in the presence of diet.
- NASH WD 6 months diet - followed by 12 weeks treatment in the presence of diet.
- ND-controls 6 months diet - followed by 12 weeks treatment in the presence of diet.

This will include GPIba transgenic C57B/6 mice and GPIba transgenic C57B/6 mice lacking the thrombin binding site, which are induced with NASH (CD-HFD and WD) that will be treated with 6 different, pre-selected anti- GPIba nanobodies, based on the publication by Mahlemir et al., 2019 Nature Medicine (group of mice n=8). Mice will be fed for 6 months with the respective NASH diets and monitored non-invasively with serum markers and ultra sound (US). At the time point at which all mice display NASH (see also Mahlemir et al., Nature Medicine 2019) the treatment with nanobodies for 12 weeks is started. Two doses of treatment will be used (high and low). After 4 and 8 weeks of treatment, non-invasive analyses are performed to follow the effects of the treatment. At the end of the treatment phase the mice are sacrificed and liver histology for NASH (e.g. H/E and special stains) is analyzed, to perform a NAS score, FACS analysis as well as histology for immune cells, fibrosis, fat deposition, perform molecular analysis for lipid metabolism, ELISA and single cell metabolomics in *situ* is performed.

One experiment for one group (e.g. humanized GPIba mouse; n=8 per group)
a) NASH CD-HFD 6 months diet - followed by 12 weeks nanobody treatment in the presence of diet.
b) NASH WD 6 months diet - followed by 12 weeks nanobody treatment in the presence of diet.
c) ND-controls 6 months diet - followed by 12 weeks nanobody treatment in the presence of diet.

Histological (H/E, Sudan red, Immunohistochemistry for B-, T-cells, T-reg. etc.), cellular (FACS), serological (ALT, AST) and molecular analysis (e.g. transcriptome, liver trigylicerides) will help to identify the nanobody candidates, that are to be used for additional experiments. At the same time the histological analyses of the most important organs (heart, lung, pancreas, kidney, gastrointestinal tract) are performed to analyze possible toxic effects.

## Claims

1. **A compound for use in the treatment of non-alcoholic fatty liver disease (NAFLD) or non-alcoholic steatohepatitis (NASH),** or of a disorder or condition associated with NAFLD or NASH, such as a disorder or condition developing from NAFLD or NASH (such as Hepatocellular Carcinoma), wherein the compound specifically binds to an ectodomain of platelet glycoprotein Ib (GPIb).

2. The compound for use according to claim 1, wherein the compound binds to a thrombin binding site, preferably to a α-thrombin binding site, wherein said binding site is located within the ectodomain of GPIbα; and/or wherein said compound binds the ectodomain of GPIbα such that upon binding between the compound and GPIbα a further binding of a thrombin (such as α-thrombin) to GPIbα is reduced or impaired, for example by sterically hindering a GPIbα-thrombin interaction and/or by changing the 3-dimensional conformation of GPIbα.

3. The compound for use according to claim 1 or 2, wherein the compound specifically binds to a leucine rich repeat containing domain in the ectodomain of GPIbα.

4. The compound for use according to any one of claims 1 to 3, wherein GPIba is human GPIbα,or a variant thereof, and preferably wherein human GPIbα comprises an amino acid sequence shown in SEQ ID NO: 1 (UniProt ref: P07359).

5. The compound for use according to claim 4, wherein the variant of GPIbα is (i) a thrombin binding fragment of GPIbα,and/or (ii) a protein having an amino acid sequence which is at least 80% identical to the amino acid sequence shown in SEQ ID NO: 1.

6. The compound for use according to any one of claims 1 to 5, wherein the compound competes with thrombin, preferably α-thrombin, for binding to the ectodomain of GPIb.

7. The compound for use according to any one of claims 1 to 6, wherein the compound does not compete with von Willebrandt factor (vWF), P-selectin, Mac-1, coagulation factor XI and/or coagulation factor XII.

8. The compound for use according to any one of claims 1 to 7, wherein the compound competes with pop/B-antibody, or with an antigen binding fragment thereof, for binding to GPIb.

9. The compound for use according to any one of claims 1 to 8, wherein the compound is an antigen binding construct selected from the group consisting of an antibody, nanobody, scFv, Fab, antibody-like molecule or other antigen binding derivative, or an antigen binding fragment thereof; and wherein the compound binds said ectodomain of GPIba, or an ectodomain of said variant of GPIba.

10. The compound for use according to any one of claims 1 to 9, wherein the treatment is a prevention of HCC in a NASH-patient at risk to develop cirrhosis and/or HCC.

11. **A pharmaceutical composition** comprising the compound recited in any one of claims 1 to 10, together with a pharmaceutically acceptable carrier and/or excipient, for a use recited in any one of claim 1 to 10.

12. **A method for producing an antibody, or antigenic binding fragment thereof,** comprising the steps of: immunizing a non-human animal with a peptide comprising, or consisting of, the ectodomain of GPIbα, and isolating from that immunized mammal an immune cell expressing such antibody.

13. **A method for identifying a compound suitable for the treatment of non-alcoholic fatty liver disease (NAFLD) or non-alcoholic steatohepatitis (NASH),** or of a disorder or condition associated with NAFLD or NASH, such as a disorder or condition developing from NAFLD or NASH, the method comprising the steps of
**(a)** Providing (x) a first cell expressing a protein or mRNA of GPIbα, or of a variant of GPIbα,or providing (y) a first test protein comprising, or (essentially) consisting of, an ectodomain of GPIbα, or of a variant of GPIbα,
**(b)** Providing a candidate compound,
**(c)** Bringing into contact the first cell or first test protein and the candidate compound, and
**(d)** Determining subsequent to step (c), either or both of:
**(i)** A binding of the candidate compound to the first cell or to the first test protein; and/or
**(ii)** A binding of the candidate compound to first cell or first test protein which binding is competitive with a thrombin protein;
wherein a binding in (i) or a competitive binding in (ii) indicates the candidate compound as suitable for the treatment of non-alcoholic fatty liver disease (NAFLD) or non-alcoholic steatohepatitis (NASH), or of a disorder or condition associated with NAFLD or NASH, such as a disorder or condition developing from NAFLD or NASH.

14. The method according to claim 13, wherein the first cell is a platelet.

15. The method according to claim 13 or 14, wherein the binding of the candidate compound to the first cell or first test protein is an interaction between the candidate compound and the ectodomain of GPIbα, preferably is a binding to a thrombin binding site, preferably to a α-thrombin binding site, wherein said binding site is located within the ectodomain of GPIbα; and/or wherein said candidate compound binds the ectodomain of GPIbα such that upon binding between the compound and GPIbα a further binding of a thrombin (such as α-thrombin) to GPIbα is reduced or impaired, for example by sterically hindering a GPIb-thrombin interaction and/or by changing the 3-dimensional conformation of GPIbα.


SEQUENCE LISTING

<110>  Deutsches Krebsforschungszentrum Stiftung des öffentlichen Rechts

<120>  TREATING NON-ALCOHOLIC STEATOHEPATITIS (NASH) AND HEPATOCELLULAR
       CARCINOMA (HCC) WITH COMPOUNDS BINDING THE ECTODOMAIN OF PLATELET
       GLYCOPROTEIN IB (GPIB) ALPHA

<130>  D31702EP

<160>  1

<170>  PatentIn version 3.5

<210>  1
<211>  654
<212>  PRT
<213>  homo sapiens

<400>  1

Met Pro Leu Leu Leu Leu Leu Leu Leu Leu Pro Ser Pro Leu His Pro
1               5                   10                  15

His Pro Ile Cys Glu Val Ser Lys Val Ala Ser His Leu Glu Val Asn
            20                  25                  30

Cys Asp Lys Arg Asn Leu Thr Ala Leu Pro Pro Asp Leu Pro Lys Asp
        35                  40                  45

Thr Thr Ile Leu His Leu Ser Glu Asn Leu Leu Tyr Thr Phe Ser Leu
    50                  55                  60

Ala Thr Leu Met Pro Tyr Thr Arg Leu Thr Gln Leu Asn Leu Asp Arg
65                  70                  75                  80

Cys Glu Leu Thr Lys Leu Gln Val Asp Gly Thr Leu Pro Val Leu Gly
                85                  90                  95

Thr Leu Asp Leu Ser His Asn Gln Leu Gln Ser Leu Pro Leu Leu Gly
            100                 105                 110

Gln Thr Leu Pro Ala Leu Thr Val Leu Asp Val Ser Phe Asn Arg Leu
        115                 120                 125

Thr Ser Leu Pro Leu Gly Ala Leu Arg Gly Leu Gly Glu Leu Gln Glu
    130                 135                 140

Leu Tyr Leu Lys Gly Asn Glu Leu Lys Thr Leu Pro Pro Gly Leu Leu
145                 150                 155                 160

Thr Pro Thr Pro Lys Leu Glu Lys Leu Ser Leu Ala Asn Asn Asn Leu
                165                 170                 175

Thr Glu Leu Pro Ala Gly Leu Leu Asn Gly Leu Glu Asn Leu Asp Thr
            180                 185                 190

Leu Leu Leu Gln Glu Asn Ser Leu Tyr Thr Ile Pro Lys Gly Phe Phe
        195                 200                 205

Gly Ser His Leu Leu Pro Phe Ala Phe Leu His Gly Asn Pro Trp Leu
    210                 215                 220

Cys Asn Cys Glu Ile Leu Tyr Phe Arg Arg Trp Leu Gln Asp Asn Ala
225                 230                 235                 240

Glu Asn Val Tyr Val Trp Lys Gln Gly Val Asp Val Lys Ala Met Thr
                245                 250                 255

Ser Asn Val Ala Ser Val Gln Cys Asp Asn Ser Asp Lys Phe Pro Val
            260                 265                 270

Tyr Lys Tyr Pro Gly Lys Gly Cys Pro Thr Leu Gly Asp Glu Gly Asp
        275                 280                 285

Thr Asp Leu Tyr Asp Tyr Tyr Pro Glu Glu Asp Thr Glu Gly Asp Lys
    290                 295                 300

Val Arg Ala Thr Arg Thr Val Val Lys Phe Pro Thr Lys Ala His Thr
305                 310                 315                 320

Thr Pro Trp Gly Leu Phe Tyr Ser Trp Ser Thr Ala Ser Leu Asp Ser
                325                 330                 335

Gln Met Pro Ser Ser Leu His Pro Thr Gln Glu Ser Thr Lys Glu Gln
            340                 345                 350

Thr Thr Phe Pro Pro Arg Trp Thr Pro Asn Phe Thr Leu His Met Glu
        355                 360                 365

Ser Ile Thr Phe Ser Lys Thr Pro Lys Ser Thr Thr Glu Pro Thr Pro
    370                 375                 380

Ser Pro Thr Thr Ser Glu Pro Val Pro Glu Pro Ala Pro Asn Met Thr
385                 390                 395                 400

Thr Leu Glu Pro Thr Pro Ser Pro Thr Thr Pro Glu Pro Thr Ser Glu
                405                 410                 415

Pro Ala Pro Ser Pro Thr Thr Pro Glu Pro Thr Ser Glu Pro Ala Pro

            420                 425                 430

Ser Pro Thr Thr Pro Glu Pro Thr Ser Glu Pro Ala Pro Ser Pro Thr
        435                 440                 445

Thr Pro Glu Pro Thr Pro Ile Pro Thr Ile Ala Thr Ser Pro Thr Ile
    450                 455                 460

Leu Val Ser Ala Thr Ser Leu Ile Thr Pro Lys Ser Thr Phe Leu Thr
465                 470                 475                 480

Thr Thr Lys Pro Val Ser Leu Leu Glu Ser Thr Lys Lys Thr Ile Pro
                485                 490                 495

Glu Leu Asp Gln Pro Pro Lys Leu Arg Gly Val Leu Gln Gly His Leu
            500                 505                 510

Glu Ser Ser Arg Asn Asp Pro Phe Leu His Pro Asp Phe Cys Cys Leu
        515                 520                 525

Leu Pro Leu Gly Phe Tyr Val Leu Gly Leu Phe Trp Leu Leu Phe Ala
    530                 535                 540

Ser Val Val Leu Ile Leu Leu Leu Ser Trp Val Gly His Val Lys Pro
545                 550                 555                 560

Gln Ala Leu Asp Ser Gly Gln Gly Ala Ala Leu Thr Thr Ala Thr Gln
                565                 570                 575

Thr Thr His Leu Glu Leu Gln Arg Gly Arg Gln Val Thr Val Pro Arg
            580                 585                 590

Ala Trp Leu Leu Phe Leu Arg Gly Ser Leu Pro Thr Phe Arg Ser Ser
        595                 600                 605

Leu Phe Leu Trp Val Arg Pro Asn Gly Arg Val Gly Pro Leu Val Ala
    610                 615                 620

Gly Arg Arg Pro Ser Ala Leu Ser Gln Gly Arg Gly Gln Asp Leu Leu
625                 630                 635                 640

Ser Thr Val Ser Ile Arg Tyr Ser Gly His Ser Leu Ser Leu
                645                 650